# EUROPÄISCHE PATENTSCHRIFT

(11) **EP 2 702 951 B1**
(45) Veröffentlichungstag und Bekanntmachung des Hinweises auf die Patenterteilung: **12.11.2014**
(21) Anmeldenummer: 12183021.0
(22) Anmeldetag: 04.09.2012
(51) Int. Cl.: A61B 17/16

(54) **Medizinisches, insbesondere chirurgisches Schiebeschaftinstrument**
Medical, in particular surgical, sliding shaft instrument
Instrument médical, en particulier chirurgical, à tige coulissante

(43) Veröffentlichungstag der Anmeldung: 05.03.2014
(73) Patentinhaber: WALDEMAR LINK GmbH & Co. KG, 22339 Hamburg (DE)
(72) Erfinder: Dmuschewsky, Klaus, 22397 Hamburg (DE)
(74) Vertreter: Raffay & Fleck

(56) Entgegenhaltungen:
- EP-A2- 1 212 983
- DE-A1- 10 061 512
- DE-A1- 19 748 369
- DE-C1- 4 316 769
- DE-U1- 20 103 630
- DE-U1-202009 002 433
- US-A1- 2003 088 268
- US-A1- 2006 116 706

## Beschreibung

Die vorliegende Erfindung betrifft ein medizinisches, insbesondere chirurgisches, Schiebeschaftinstrument gemäß den Merkmalen des Oberbegriffs des Anspruches 1.

Derartige Schiebeschaftinstrumente zeichnen sich dadurch aus, dass sie ein typischerweise längserstrecktes Schaftelement aufweisen, an welchem ein Schieber in axialer Richtung des Schaftelementes verschiebbar angeordnet ist. Schieber und Schaftelement sind typischerweise über eine Führung miteinander verbunden, die über einen weiten Bereich der möglichen Relativstellungen des Schiebers und Schaftelementes zueinander eine Trennung dieser beiden Elemente nicht gestattet, die Längsbewegung des Schiebers führt und gegenüber weiteren Relativbewegungen des Schiebers relativ zu dem Schaftelement sichert. Derartige Führungen können beispielsweise vermittels in eine Schwalbenschwanznut an einem der Elemente eingreifender Keilstücke oder eines Keilstückes an dem anderen der genannten Elemente realisiert sein.

Ferner umfassen solche Schiebeschaftinstrumente einen ersten und einen zweiten Griffarm, wobei der zweite Griffarm in Bezug auf den ersten Griffarm schwenkbar gelagert ist. Die Lagerung kann dabei unmittelbar an dem ersten Griffarm, aber auch an einem anderen der Teile bzw. Elemente des Schiebeschaftinstrumentes, beispielsweise an dem Schaftelement, erfolgen. Es kommt hierbei lediglich darauf an, dass ein Verschwenken der beiden Griffarme relativ zueinander möglich ist. Bei den Griffarmen, die auf diesem Fachgebiet häufig auch als "Griffbranchen" bezeichnet werden, handelt es sich um solche Griffstücke zum Handhaben der Schiebeschaftvorrichtung, die zugleich auch eine Hebelarm-Funktion erfüllen. Sie können beispielsweise vergleichbar den Griffarmen von Schereninstrumenten oder dgl. ausgestaltet sein.

Die Schiebeschaftinstrumente, die allgemein Gegenstand der hierin beschriebenen Erfindung sind, weisen typischerweise an einem distalen Ende des Schaftelementes einen Funktionsabschnitt auf, der in seiner Funktionalität auch das distale Ende des auf dem Schaftelement axial verschiebbaren Schiebers mit einbezieht. So können beispielsweise das distale Ende des Schaftelementes und das distale Ende des Schiebers ausgestaltet sein, um zwei Stanzbacken einer Stanze zu bilden, es können Schneiden vorgesehen sein, die das Schiebeschaftinstrument zu einem Schneidinstrument bzw. einer Schneid- oder Kneifzange machen, es können an den distalen Enden entsprechende Greifelemente angeordnet sein, um aus dem Schiebeschaftelement einen Greifer werden zu lassen und dgl. Hier kommen grundsätzlich alle möglichen Ausgestaltungsformen eines Instrumentes in Betracht, welches über eine Schiebeschafttechnik bedienbar ist und welches insbesondere in vorteilhafter Weise den Einsatz eines verlängerten Schaftes voraussetzt bzw. über einen solchen Schaft für den Einsatz Vorteile nimmt. Typischerweise werden derartige Schiebeschaftinstrumente im Bereich der minimalinvasiven Eingriffe Verwendung finden, beispielsweise um über natürliche Körperöffnungen eingeführt Zugang zu im Inneren des Körpers liegenden Bereichen zu ermöglichen, beispielsweise für das Ausräumen von Nasennebenhöhlen oder die Entfernung von Polypen durch die Nasenöffnungen oder aber auch operative Eingriffe im Bereich des Abdomens durch kleine Zugangsöffnungen und -schnitte oder aber Eingriffe im Bereich der Wirbelsäule von der Bauchseite her ebenfalls über einen minimalinvasiven klein dimensionierten Zugang. Weitere Anwendungsmöglichkeiten sind aber ebenfalls denkbar, z.B. als Halter für Tupfer oder dgl. im Zuge "herkömmlicher" Eingriffe und Operationen, wo es von Vorteil ist, den eigentlichen Arbeitsbereich, in welchem der Tupfer eingesetzt werden soll, nicht durch groß dimensionierte Halter zu verdecken und die Sicht einzuschränken und deshalb entsprechende Gerätschaften, wie beispielsweise Tupfer am Ende von langen und schmalen Instrumentenschäften anzuordnen.

Für eine nach Gebrauch erforderliche Reinigung und Desinfektion derartiger Schiebeschaftinstrumente sind diese in den meisten Fällen zerlegbar gestaltet. Hierfür ist dann eine Möglichkeit geschaffen, den Schieber - typischerweise in einer proximalen Endstellung - von dem Schaftelement zu lösen und abzunehmen. In dieser proximalen Endstellung entfällt also die Verriegelung zwischen Schieber und Schaftelement gegen Verlagerungen quer zur axialen Relativbewegungsrichtung. Darüber hinaus ist in einer solchen Endstellung eine Möglichkeit zu schaffen, den Schieber aus einer Kupplung mit dem zweiten Griffarm zu lösen.

Vergleichbare Schiebeschaftinstrumente sind beispielsweise offenbart in der DE 197 48 369 A1. Bei dem dort offenbarten Instrument ist an dem ersten Griffarm ein verdrehbares Riegelelement angeordnet, welches in einer Freigabestellung eine Weiterbewegung des Schiebers über die proximale Endstellung hinaus erlaubt, wobei sich dann der Querstift aus dem in dem zweiten Griffarm ausgebildeten Schlitz heraus erlaubt.

Ein weiteres vergleichbares Schiebeschaftinstrument ist in der DE 20 2008 001 675 U1 offenbart und beschrieben. Dort ist in einer vergleichbaren konstruktiven Lösung ebenfalls ein Verriegelungselement am ersten Griffarm angeordnet, welches den Schieber in einer proximalen Endposition stoppt, bei Freigabe eine weitere in proximaler Richtung weisende axiale Längsbewegung des Schiebers gegenüber dem Schaftelement gestattet. Bei einer solchen weiteren Bewegung wird eine Führungsnase an dem Schieber in eine Position in einer entsprechenden Führungsnut an dem Schaftelement verbracht, in welcher es von dem Schaftelement getrennt werden kann, dabei auch der Querstift an dem Schieber aus dem Schlitz des zweiten Griffarms gleitet.

In der DE 100 61 512 A1 ist ein vergleichbares Schiebeschaftinstrument offenbart, bei welchem ebenfalls ein Riegelhebel den Schieber in einer proximalen Endposition stoppt, wenn der Riegelhebel in einer Sperrstellung befindlich ist. Wird der Riegelhebel aus der Sperrstellung in eine Freigabestellung bewegt, ist eine über die proximale Endstellung hinausgehende Bewegung des Schiebers relativ zu dem Schaftelement ermöglicht, bis hin zu einer Position, in der der Querstift aus dem Führungsschlitz entnommen werden kann. Eine federbelastete Sperrkugel, die in einem den Schlitz des zweiten Griffarmes begrenzenden Schenkel angeordnet ist, bildet eine gewisse Verrastung des Querstiftes in dem Schlitz, die bei Übersteigen einer Lösekraft überwunden werden kann.

Bei einem weiteren Schiebeschaftinstrument, welches in der DE 297 18 969 U1 offenbart ist, wird die proximale Endstellung des Schiebers durch ein aus zwei jeweils mit einem der Griffarme verbundenen Gestängeelementen gebildetes, lösbar miteinander verbundenes Feder- und Wegbegrenzergestänge gebildet. Wird die lösbare Verbindung zwischen den Gestängeelementen getrennt, kann der Schieber weiter in proximaler Richtung bewegt und der Querstift aus dem Schlitz gelöst werden zum Trennen der Elemente für ein Zerlegen derselben.

Es hat sich nun herausgestellt, dass die vorbekannten Konstruktionen und Mechanismen für die lösbare Verbindung der Komponenten Schaftelement, Schieber und Griffarm in der Handhabung umständlich sind, was einesteils das Bedienen, anderenteils die Reinigung und Sterilisierung betrifft. Zum einen besteht bei im Bereich der Griffarme angeordneten Riegelschaltern insbesondere dann, wenn diese als Schiebeschalter ausgebildet sind, die Gefahr einer unbeabsichtigten Betätigung während des Gebrauches, wenn beispielsweise mit dem Daumen einer das Schiebeschaftinstrument betätigenden Hand ein zusätzlicher Druck in einem oberen Abschnitt des Instrumentes am proximalen Ende des Schaftelementes bzw. im oberen Bereich eines Griffarmes aufgebracht werden muss. Wird dann eine Bewegung des Schiebers in Richtung des proximalen Endes ausgeführt und gerät diese über die Endstellung, in der Schieber und Schaftelement noch verbunden sind, hinaus, kann das Instrument unbeabsichtigt während des Einsatzes zerlegt werden mit entsprechenden negativen Folgen und Beeinträchtigungen des Ablaufes des medizinischen Eingriffes, bei dem das Schiebeschaftinstrument zum Einsatz kommt. Darüber hinaus bieten sämtliche derartige Riegelschalter, -schieber oder Drehriegel weitere kleine Zwischenräume, in welche während des medizinischen Eingriffes und Einsatzes des Schiebeschaftinstrumentes Verunreinigungen mit Blut, Gewebeteilen, Knochenspäne oder dgl. eindringen können und welche naturgemäß nur schwer zugänglich und damit nur mit erheblichem Aufwand und oft sogar nur unzureichend zu reinigen und anschließend zu sterilisieren sind.

Hier eine Vereinfachung zu schaffen und ein gattungsgemäßes Schiebeschaftinstrument anzugeben, welches einfach im Handling und gut und rückstandsfrei zu reinigen und einfach zu sterilisieren ist, ist Aufgabe der vorliegenden Erfindung.

Erfindungsgemäß besteht die Lösung dieser Aufgabe darin, ein medizinisches, insbesondere chirurgisches, Schiebeschaftinstrument der eingangs genannten Art so zu gestalten, dass ein erster Schenkel der den ersten Schlitz begrenzenden Schenkel als quer zu seiner Längserstreckungsrichtung von dem zweiten der Schenkel nach außen weg bewegbare Federzunge gebildet ist, wobei in der proximalen Endstellung der zweite Griffarm derart positioniert ist, dass der Querstift in dem ersten Schlitz liegt und in einer im Wesentlichen senkrecht von der axialen Linie des Schaftelementes in Richtung des Schiebers weisenden Löserichtung von dem ersten als Federzunge gebildeten Schenkel zumindest teilweise überdeckt und zurückgehalten ist, durch Aufbringen einer Lösekraft in der Löserichtung und gegen die Federkraft der Federzunge gerichteten Verlagerung des Endes des ersten Schenkels aus dem ersten Schlitz lösbar ist und wobei an dem proximalen Ende des Schiebers ein Ansatz angeordnet ist, der in der proximalen Endstellung über ein proximales Ende des Schaftelementes hinausragt und über den eine in Löserichtung wirkende Lösekraft auf den Schieber aufbringbar ist.

Bei der wie oben beschriebenen Ausgestaltung ist zunächst ein wichtiges Element, dass der ersten Schlitz nicht etwa von starren Schenkeln begrenzt ist, dass vielmehr der erste Schenkel, zumindest dieser, in einer Querrichtung bewegbar als Federzunge ausgebildet ist. Dieser Ausgestaltung ermöglicht es, eine Arretierung der Einzelteile des zerlegbaren Schiebeschaftinstrumentes zu erzielen, ohne hierfür etwa weitere Hilfsmittel wie Sperrschieber oder sonstige Riegelelemente einsetzen zu müssen, welche neben möglichen Fehlbedienungen insbesondere auch die erwähnten Probleme bei der nach Gebrauch des Schiebeschaftinstrumentes durchzuführenden Reinigung und Sterilisierung aufwerfen. Weiterhin wichtig und erfindungsrelevant ist die besondere Anordnung und Lage der Elemente des Querstiftes und des ersten Schlitzes zueinander in der proximalen Endstellung. Denn durch diese ist im zusammengefügten Zustand des Schiebeschaftinstrumentes der Schieber weiterhin gesichert und gehalten, nämlich zurückgehalten durch den ersten Schenkel des ersten Schlitzes und seine Federkraft. Erst beim Überwinden einer solchen Federkraft kann der erste Schenkel nach außen weggedrückt, der ersten Schlitz gespreizt werden, so dass der Querstift in der proximalen Endstellung aus dem ersten Schlitz gedrückt und der Schieber, der in dieser proximalen Endstellung keine weitere Verbindung mit dem Schaftelement aufweist, abgehoben und von den restlichen Elementen gelöst werden kann. Dabei ist er zum Einen in vorteilhafter Weise nicht erforderlich, den Schieber über die proximale Endstellung hinaus in Richtung des proximalen Verstellweges in eine Lösestellung zu stellen, wie dies einige der Schiebeschaftinstrumente nach dem Stand der Technik erfordern. Ferner ist die besondere Anordnung und geometrische Abstimmung der Elemente erster Schenkel, erster Schlitz und Querstift von Relevanz. In der proximalen Endstellung muss nämlich der Querstift so weit in Richtung des offenen Endes des ersten Schlitzes verschoben sein, dass dieser zwar von dem ersten den ersten Schlitz begrenzenden Schenkel noch zumindest teilweise überdeckt wird, dies aber nur insoweit, als dass mit einem gegen die Rückstellkraft der Federzunge, die den ersten Schenkel bildet, gerichteten Lösekraft, dieser erste Schenkel beiseite gedrückt werden kann, so dass der Weg für den Querstift aus dem offenen Ende des ersten Schlitzes frei wird. Um eine solche Lösekraft ohne zusätzliches Werkzeug aufzubringen ist der erfindungsgemäße Ansatz vorgesehen. Dieser ist an einer in der proximalen Endstellung des Schiebers über das proximale Ende des Schaftelementes hinausstehenden Verlängerung bzw. an einem solchen Fortsatz gebildet. Eine rückwärtige, über das proximale Ende des Schaftelementes in der proximalen Endstellung des Schiebers hinausragende Verlängerung hat dabei den Vorteil, dass sie bei einer Handhabung des erfindungsgemäßen Schiebeschaftinstrumentes mit dem Daumen einer dieses Instrument bedienenden Hand einfach betätigt werden kann. So lässt sich eine einfache einhändige Zerlegbarkeit erzielen, wobei die zweite Hand einer Bedienperson frei ist, um z.B. den gelösten Schieber zu ergreifen und abzulegen.

Mit Vorteil weist der erste Schlitz an seinem offenen Ende eine V- oder keilförmige Aufweitung auf. Diese ist insbesondere so gestaltet, dass in der proximalen Endstellung des Schiebers, wenn dieser auf das Schaftelement beim Zusammenfügen des Schiebeschaftinstrumentes aufgesetzt ist, sein Bereich, in dem der Querstift angeordnet ist, in diese V- oder keilförmige Aufweitung eingesetzt ist, der Querstift durch Aufbringen einer Druckkraft unter Überwindung der Federkraft des als Federzunge ausgebildeten ersten Schenkels diesen ersten Schenkel unter Aufweitung des ersten Schlitzes abspreizt und in den tiefer liegenden Schlitzbereich gelangt, bis der erste Schenkel in seine Normalposition zurückfedert, der Querstift also in der gehaltenen Stellung einrastet bzw. einschnappt.

Um bei einer Auslenkung des als Federzunge ausgebildeten ersten Schenkels, also einer Belastung der dadurch gebildeten Feder, die Gefahr einer Rissbildung am Schlitzgrund des ersten Schlitzes deutlich zu verringern, kann, wie gemäß einer vorteilhaften Weiterbildung der Erfindung vorgesehen, am Grund des ersten Schlitzes zwischen den Schenkeln eine Schlitzaufweitung vorgesehen sein, die insbesondere einen kreisabschnittsförmigen Querschnitt aufweisen kann. Je grö-ßer der Radius einer solchen kreisförmig gestalteten Schlitzaufweitung ist, desto geringer ist die Gefahr einer Rissbildung an dieser Stelle.

Grundsätzlich ist es möglich, dass auch der zweite Schenkel ein als Federzunge gebildeter Schenkel ist. Allerdings wird in einer vorteilhaften Ausführungsform der Erfindung bevorzugt, dass dieser Schenkel ein starrer Schenkel ist. Insbesondere kann mit einem solchen starren Schenkel eine größere Kraft auf den in dem ersten Schlitz geführten Querstift übertragen werden, so dass mit Vorteil dieser zweite, starre Schenkel derjenige Schenkel ist, der bei einer Betätigung in einer Bewegungsrichtung eines klemmenden oder schneidenden Vorganges eine Kraftübertragung auf den Querstift bewirkt.

Insgesamt wirken bei einer bevorzugten Ausgestaltungsform der Erfindung die den ersten Schlitz begrenzenden Schenkel dergestalt, dass der zweite Schenkel beim Betätigen der Griffarme in eine erste Betätigungsrichtung auf den Querstift eine Kraft zum Bewegen des Schiebers in Richtung der distalen Endstellung überträgt, während der erste Schenkel beim Betätigen der Griffarme in eine zweite Betätigungsrichtung auf den Querstift eine Kraft zum Bewegen des Schiebers in Richtung der proximalen Endstellung überträgt. Bei dieser Umsetzung ist also insbesondere bei der Bewegung des Schiebers in Richtung der distale Endstellung eine höhere Kraft zu erwarten, weil dies beispielsweise eine Stanzrichtung, eine Schneidrichtung oder eine Klemmrichtung für Greifwerkzeuge ist.

Bei dem erfindungsgemäßen Schiebeschaftinstrument ist gemäß einer vorteilhaften Ausgestaltung in die Oberfläche des zweiten Griffarmes auf dessen dem ersten Griffarm zugewandten Seite eine Fingermulde eingeformt. Diese Fingermulde gibt ein definiertes Widerlager für beispielsweise den Zeigefinger einer Betätigungshand, wenn mit einem anderen Finger, insbesondere dem Daumen, eine Lösekraft auf den Ansatz aufgebracht wird. Sie ist mit anderen Worten eine der Ergonomie und Bedienerfreundlichkeit zuträgliche Ausgestaltung.

Insbesondere können, auch wenn dies nicht zwingend erforderlich ist, an den freien Enden der Griffarme jeweils eine Fingeröse ausgebildet sein. In diese können beim Bedienen des Schiebeschaftinstrumentes beispielsweise der Daumen und der Zeigefinger oder der Daumen und der Mittelfinger einer das Instrument betätigenden Bedienhand eingeführt werden, so dass sowohl in einer ersten Betätigungsrichtung, in welcher die beiden Griffarme aufeinander zu bewegt werden, als auch in einer zweiten Betätigungsrichtung, bei der die Griffarme voneinander weg bewegt und gespreizt werden, entsprechende Betätigungskräfte einfach und zuverlässig auf die Griffarme übertragen werden.

Für eine weiter verbesserte Reinigung nach Gebrauch des Schiebeschaftinstrumentes ist es von Vorteil, wenn, wie gemäß einer weiteren vorteilhaften Weiterbildung der Erfindung vorgesehen, der zweite Griffarm lösbar mit dem ersten Griffarm und/oder dem Schaftelement verbunden ist. Diese lösbare Verbindung kann insbesondere durch die Verbindung des Schiebers mit dem Schaftelement und Festlegung des Schiebers mit dem Querstift in dem ersten Schlitz des zweiten Griffarms gesichert werden, so dass nach Lösen des Schiebers von dem Schaft-element und dem zweiten Griffarm letztgenannter einfach von dem ersten Griffarm bzw. dem Schaftelement gelöst werden kann. Hierzu kann der zweite Griffarm beispielsweise einen ein freies Ende aufweisenden Drehzapfen umfassen, der mit seinem freien Ende in eine entsprechende Drehlageraufnahme an dem Schenkelelement bzw. dem ersten Griffarm eingeführt wird und entsprechend zum Lösen des zweiten Griffarms aus dieser entnommen werden kann.

Eine bevorzugte Ausgestaltungsform des Schiebeschaftinstrumentes nach der Erfindung ist in einer Schere bzw. einem zangenartigen Schneid- bzw. Kneifinstrument zu sehen. Dabei kann an dem distalen Ende des Schaftelementes eine erste feststehende Schneide ausgebildet sein und kann ferner eine relativ zu der ersten Schneide bewegbare zweite Schneide angeordnet sein, die mit dem Schieber in einer solchen Weise gekoppelt ist, dass sich bei einer Verlagerung des Schiebers in axialer Richtung die Schneiden aufeinander zu bzw. voneinander weg bewegen. Selbstverständlich kann das erfindungsgemäße Schiebeschaftinstrument aber auch in anderen Werkzeugvarianten realisiert sein, so z.B. als Stanze, als Greifer und dgl. Werkzeug, wie eingangs der allgemeinen Beschreibung bereits ausgeführt.

Gemäß einer weiteren vorteilhaften und möglichen Ausgestaltung des erfindungsgemäßen Schiebeschaftinstrumentes kann dieses in dem Bereich, in welchem der erste Schlitz in dem zweiten Griffarm angeordnet ist, einen zweiten endseitig offenen Schlitz aufweisen, welcher zweite Schlitz sich zwischen dem ersten Schenkel und einem dritten Schenkel erstreckt. Dabei bewegt sich der erste Schenkel, wenn dieser quer zu seiner Längserstreckungsrichtung von dem zweiten Schenkel nach außen weg bewegt wird, zugleich auf den dritten Schenkel zu, wobei der dritte Schenkel einen den Bewegungsweg des ersten Schenkels begrenzenden Anschlag bildet. Eine solche Ausgestaltung hilft zu verhindern, dass der als Federzunge ausgebildete erste Schenkel bei einer Auslenkung infolge eines Löse- oder Verbindungsvorganges des Schiebers mit dem Schaftelement übermäßig weit ausgelenkt wird und zu brechen droht bzw. an seinem Schlitzgrund eine Rissbildung erfährt. Eine entsprechende überweite Auslenkung des als Federzunge ausgebildeten Schlitzes wird durch den dritten Schenkel, der einen Anschlag bildet, verhindert. Der zweite Schlitz limitiert mit seiner Schlitzweite den Bewegungsweg des ersten Schenkels bei seiner Auslenkung.

Der bei der oben bezeichneten möglichen Ausgestaltungsform vorgesehene zweite Schlitz kann mit Vorteil parallel zu dem ersten Schlitz verlaufen und sich im Wesentlichen über eine gleiche Länge in das Material des zweiten Griffarmes hinein erstrecken. Diese parallele Schlitzführung und Erstreckung über im Wesentlichen die gleiche Länge in das Material des zweiten Griffarmes hinein ergibt vor allem einen ersten, als Federzunge ausgebildeten Schenkel mit gleichmäßiger Materialstärke und einem beidseits auf gleichem Niveau (bestimmt durch den Schlitzgrund des jeweils angrenzenden ersten bzw. zweiten Schlitzes) gelegenen Anlagerungspunkt. Mit Vorteil kann bei dieser Ausgestaltungsform der dritte Schenkel weiterhin ein starrer Schenkel sein, also ohne eine Federwirkung. Damit ist vor allem auch sichergestellt, dass dieser den Anschlag bildende Schenkel nicht etwa ebenfalls bei einer überhöhten Kraftaufbringung federnd ausweicht, sondern als Anschlag starr und "unausweichlich" stehen bleibt.

Insoweit in der vorstehenden Beschreibung allgemein auf einen "ersten Schlitz" Bezug genommen worden ist, der für die Erfindung wesentlich ist und strukturell gegeben sein muss, so ist damit nicht etwa gemeint, dass ein erfindungsgemäßes Schiebeschaftinstrument in jeder möglichen Ausgestaltungsform mehr als etwa nur einen, nämlich den ersten Schlitz, aufzuweisen hat. Die Bezeichnung "erster Schlitz" wurde lediglich zur besseren Unterscheidung zu dem in einem speziellen Ausführungsbeispiel vorgesehenen, wie vorstehend beschrieben ausgebildeten "zweiten Schlitz" eingeführt.

In einer weiteren Ausführungsvariante kann der Längsschlitz in einem seinem freien, offenen Ende nahegelegenen Bereich eine kreisförmige Aufweitung aufweisen, deren Durchmesser dem Durchmesser des Querstifts entspricht. In einer solchen Aufweitung kann der Querstift einrasten, so dass hierdurch bestimmte Längspositionen des Schiebers auf dem Schaftelement als "Rastpositionen" vorgegeben sind, das Schiebeschaftinstrument in solchen Positionen verrastet.

Weitere Vorteile und Merkmale der Erfindung ergeben sich aus der nachfolgenden Beschreibung möglicher Ausführungsbeispiele der Erfindung anhand der beigefügten Figuren. Dabei zeigen:
- Fig. 1: eine schematische Ansicht eines ersten eines erfindungsgemäßen Schiebeschaft-instrumentes mit in der proximalen Endstellung befindlichem Schieber;
- Fig. 2: in vergrößerter Darstellung und teilgeschnitten einen Ausschnitt des Schiebeschaftinstrumentes nach Fig. 1 mit in der distalen Endstellung befindlichem Schieber und in einer Ausgestaltung nach einem ersten Ausführungsbeispiel;
- Fig. 3: einen der Fig. 2 vergleichbaren vergrößerten und teilgeschnittenen Ausschnitt des ersten Ausführungsbeispiels mit dem Schieber in der proximalen Endstellung;
- Fig. 4: eine der Fig. 2 vergleichbare Darstellung und teilgeschnitten einen Ausschnitt des Schiebeschaftinstruments nach Fig. 1 mit in der distalen Endstellung befindlichem Schieber und in einer Ausgestaltung nach einem zweiten Ausführungsbeispiel;
- Fig. 5: einen der Fig. 3 vergleichbaren vergrößerten und teilgeschnittenen Ausschnitt des zweiten Ausführungsbeispiels mit dem Schieber in der proximalen Endstellung;
- Fig. 6: einen größeren Ausschnitt des Schiebeschaftinstruments in der Ausgestaltung des zweiten Ausführungsbeispiels mit dem Schieber in der proximalen Endstellung, in der auch eine Führungsverbindung zwischen Schieber und Schaftelement erkennbar ist;
- Fig. 7: in zwei Darstellungen a) und b) die Situation beim Trennen des Schiebers vom Schaftelement bei dem zweiten Ausführungsbeispiel unter Einbeziehung der Trennung der Führungsverbindung und
- Fig. 8: eine dreidimensionale Ansicht des Schiebeschaftinstruments nach dem zweiten Ausführungsbeispiel mit von dem Schaftinstrument abgehobenem Schieber.

In den Figuren ist in zwei möglichen Ausführungsbeispielen ein erfindungsgemäßes Schiebeschaftinstrument schematisch dargestellt und wird nachfolgend - in beiden Ausgestaltungsvarianten - erläutert. Dabei sind die Figuren bloße Schemazeichnungen und keinesfalls maßstabsgerecht oder im Detail vollständig. Sie dienen, wie erwähnt, lediglich der Erläuterung der möglichen Umsetzung der wesentlichen Merkmale des erfindungsgemäßen Schiebeschaftinstrumentes in einem veranschaulichenden Ausführungsbeispiel. In den Figuren sind gleiche bzw. gleichartige Elemente mit gleichen Bezugsziffern versehen, auch für die unterschiedlichen Ausführunsgbeispiele.

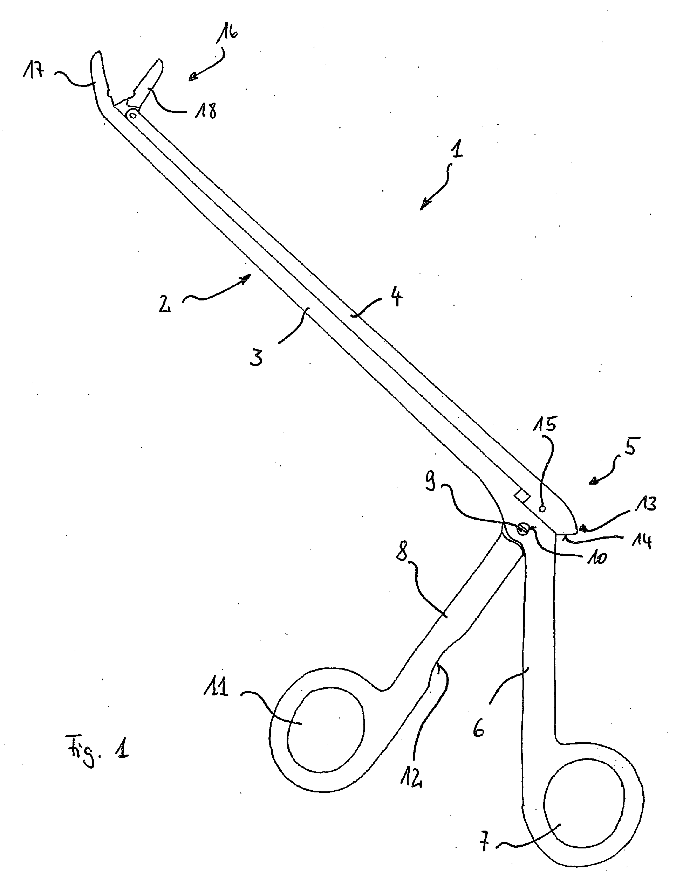

In Fig. 1 ist ein erfindungsgemäßes Schiebeschaftinstrument 1 dargestellt, in dieser Ausführungsvariante in Form eines sogenannten Rongeurs. Dieses Schiebeschaftinstrument 1 weist einen langgestreckten, gerade verlaufenden Schaft 2 auf, der ein Schaftelement 3 und einen relativ zu diesem Schaftelement 3 in axialer Richtung des Schaftes 2 bzw. des Schaftelementes 3 bewegbaren Schieber 4 enthält. An einem proximalen Ende 5 ist mit dem Schaftelement 3 fest, insbesondere einstückig, verbunden und gegenüber der Längsrichtung bzw. axialen Richtung des Schaftelementes 3 abgewinkelt ein erster Griffarm 6 angeformt. An seinem freien Ende weist dieser erste Griffarm 6 eine Fingeröse 7 auf.

Ein zweiter Griffarm 8 ist relativ zu dem ersten Griffarm 6 verschwenkbar angeordnet. Dazu ist dieser mit einem Schwenklagerstift 9 in einer im Übergangsbereich zwischen dem Schaftelement 3 und dem ersten Griffarm 6 als Bohrung gebildeten Stiftaufnahme 10 gelagert. Auch der zweite Griffarm 8 weist an seinem freien Ende einer Fingeröse auf, die mit 11 bezeichnet ist. Darüber hinaus ist in der Oberfläche des zweiten Griffarmes 8 auf einer dem ersten Griffarm 6 zugewandten Seite eine Fingermulde 12 geformt. Diese dient zum Abstützen beispielsweise eines Zeige- oder Mittelfingers der Betätigungshand, wenn mit dem Daumen der Betätigungshand in der Fingeröse 7 eine Spreizbewegung der Griffarme 6, 8 durchgeführt wird oder aber auch in später noch näher zu beschreibender Weise der Schieber 4 von dem Schaftelement 3 und dem zweiten Griffarm 8 gelöst wird.

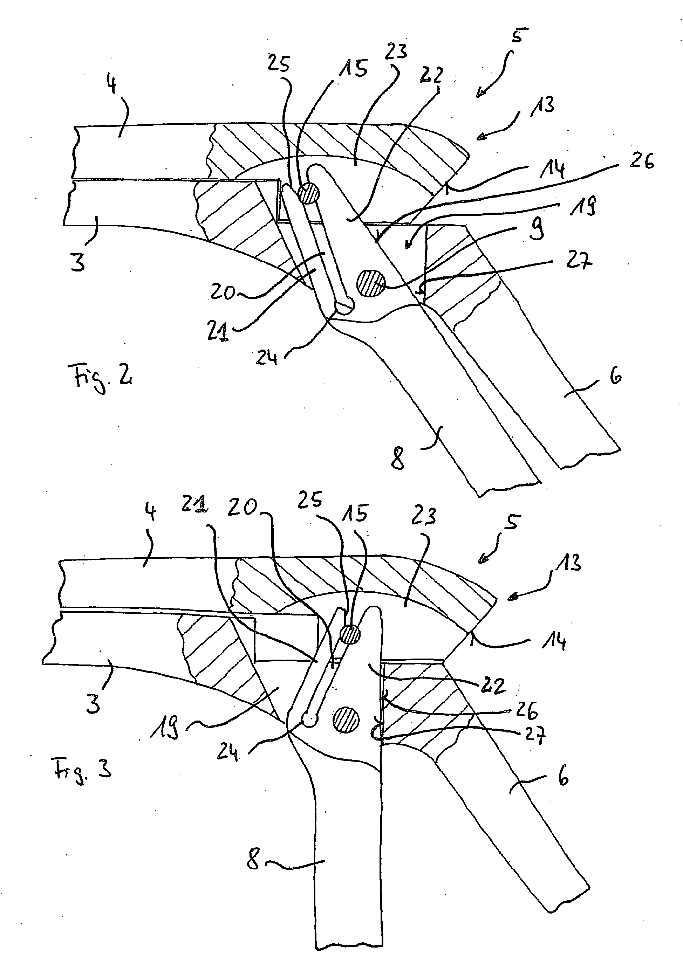

Zu erkennen ist auch, dass in seiner in Fig. 1 dargestellten proximalen Endstellung der Schieber 4 an seinem rückwärtigen bzw. proximalen Ende eine über das proximale Ende des Schaftelementes 3 bzw. über den Verlauf des ersten Griffarmes 6 überstehenden Fortsatz 13 aufweist mit einem als Angriffsfläche ausgebildeten Ansatz 14. Dieser Ansatz 14 und seine Bedeutung werden nachfolgend anhand der Beschreibung insbesondere der Fig. 3 noch näher erläutert.

Zu erkennen ist auch ein an dem Schieber 4 angeordneter Querstift 15, der mit einer die Oberfläche des Schiebers durchdringenden Endseite bzw. Stirnseite hier zu erkennen ist.

An seinem distalen Ende 16 weist das Schiebeschaftinstrument 1 zwei gemeinsam ein Schneidwerkzeug bildende Räumschneiden 17 und 18 auf. Die erste Räumschneide 17 ist fest und einstückig mit dem Schaftelement 3 verbunden, gegenüber diesem abgewinkelt. Die zweite Räumschneide 18 ist mit dem Schaftelement 3 in einem Schwenklager verbunden und ferner an dem Schieber 4 angelenkt. Auf dieser Weise wird durch eine axiale Längsbewegung des Schiebers 4 entlang des Schaftelementes 3 eine Schließ- bzw. Öffnungsbewegung der Räumschneiden 17 und 18 erreicht. Wird der Schieber 4 entlang des distalen Endes 16 bewegt, so schließen die Räumschneiden 17 und 18 und können Knochen- oder Knorpelmaterial durchtrennen. Bei umgekehrter Bewegungsrichtung des Schiebers 4 öffnen die Räumschneiden 17, 18.

Die axiale Bewegung des Schiebers 4 in Richtung des distalen Endes 16 wird bewirkt durch ein Zusammenziehen der beiden Griffarme 6 und 8. Über eine anhand der nachfolgenden Figuren noch näher zu beschreibende Verbindung des zweiten Griffarmes 8 mit dem Querstift 15 und damit mit dem Schieber 4 wird eine in Richtung des distalen Endes 16 weisende Bewegung des Schiebers 4 in axialer Richtung entlang dem Schaftelement 3 bewirkt. Durch das Spreizen der Griffarme 6, 8 wird über den gleichen Mitnahmemechanismus eine Bewegung des Schiebers 4 in Richtung des proximalen Endes 5 erzielt.

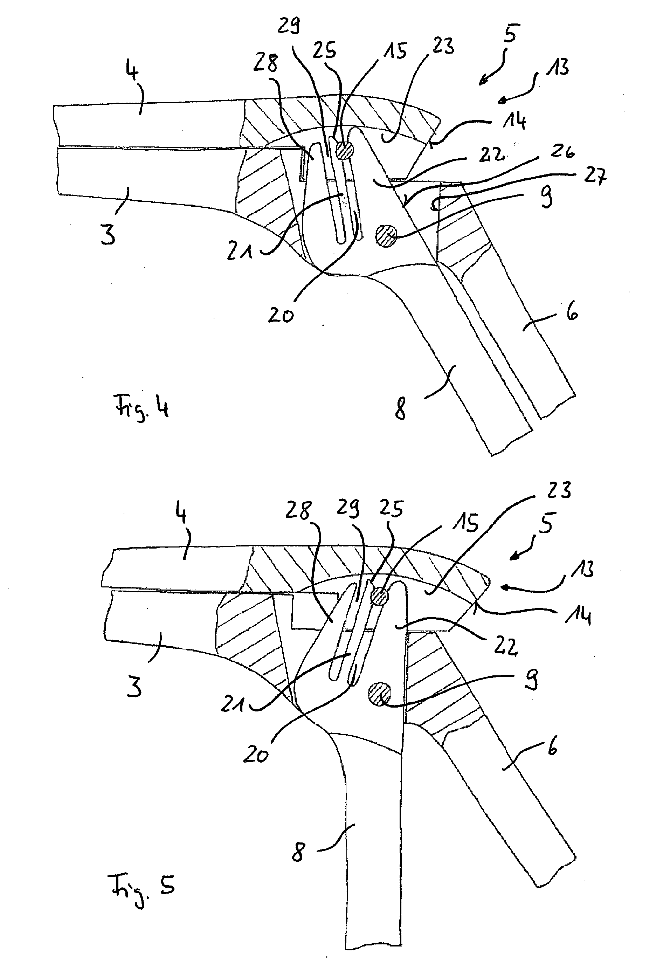

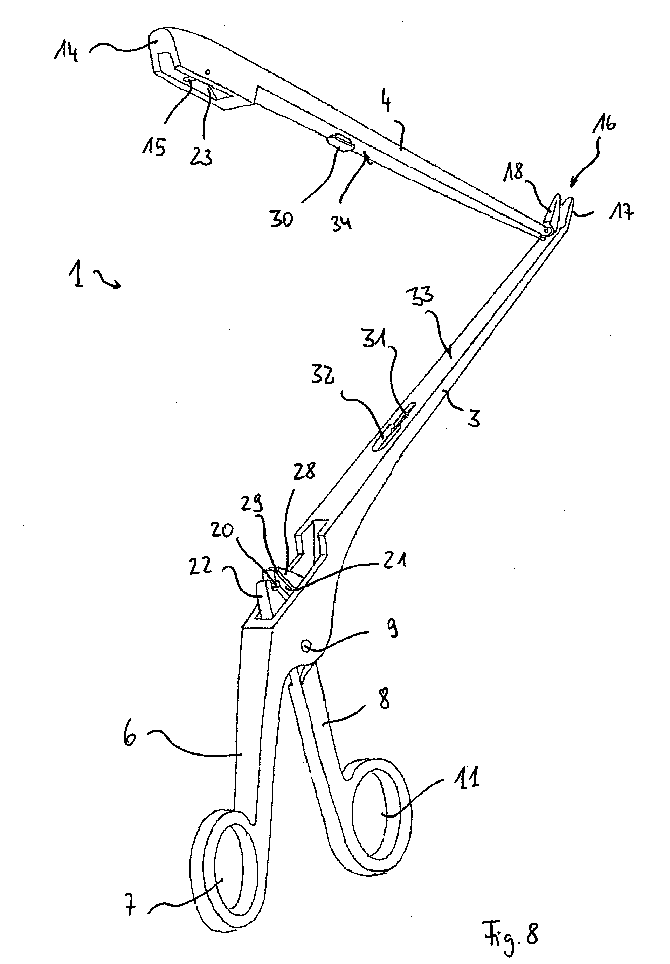

Zwischen dem Schaftelement 3 und dem Schieber 4 ist eine Führung ausgebildet, die insbesondere durch eine auf der dem Schieber 4 zugewandten Seite des Schaftelementes 3 eingebrachte, in axialer Richtung verlaufenden Schwalbenschwanznut und einen korrespondierend geformten Nutenstein auf der dem Schaftelement 3 zugewandten Oberfläche des Schiebers realisiert sein kann. Dabei ist diese Führung so ausgebildet, dass in der in Fig. 1 gezeigten proximalen Endstellung des Schiebers 4 diese Führung trennbar ist, also beispielsweise der Nutenstein aus der Schwalbenschwanznut entfernbar ist, durch eine entsprechende Aufweitung der Nut. Diese Elemente sind hier nicht gezeigt, lassen sich aber in der Darstellung des zweiten Ausführungsbeispiels der Erfindung in den Figuren 4 bis 8 erkennen. Diese Führung kann dabei auch gestaltet sein wie die in der DE 20 2008 001 675 U1 oder auch die in der DE 20 2012 001 348 U1 offenbarten und beschriebenen Führungen.

In den teilweise geschnittenen Ausschnittsdarstellungen der Figuren 2 und 3 ist das Zusammenwirken und sind weitere Einzelteile der Bewegungsübertragungsmimik und zugleich lösbaren Verbindung zwischen dem zweiten Griffarm 8 und dem Schieber 4 in einem ersten Ausführungsbeispiel dargestellt. In Fig. 2 ist eine Situation gezeigt, in der sich der Schieber 4 in seiner distalen Endstellung befindet, in Fig. 3 befindet sich der Schieber 4 in seiner proximalen Endstellung. Nachfolgend wird zunächst, sofern nicht explizit die betroffene Figur genannt wird, zugleich auf beide Figuren Bezug genommen.

Dort ist zu erkennen, wie durch eine im Übergangsbereich zwischen Schaftelement 3 und erstem Griffarm 6 gebildete Durchführung 19 ein Ende des zweiten Griffarmes 8 geführt ist, wobei in der Durchführung 19 der Schwenklagerstift 9 angeordnet ist und eine relativ zu dem ersten Griffarm 6 und dem Schaftelement 3 ortsfeste Schwenkachse definiert. Erkennbar ist hier auch ein an dem der (in den Figuren nicht dargestellten) Grifföse 11 gegenüberliegenden Ende des zweiten Griffarmes 8 ausgebildeter Schlitz 20. In diesem Schlitz 20, der seitlich von einem ersten Schenkel 21 und einem zweiten Schenkel 22 begrenzt ist, ist der Querstift 15 gefangen, so dass sich über diese Anordnung eine gelenkige Verbindung zwischen dem zweiten Griffarm 8 und dem Schieber 4 ergibt. Um dieser gelenkigen Verbindung Raum zu geben ist am proximalen Ende 5 in dem Schieber 4 eine im Querschnitt bogenförmige Ausnehmung bzw. ein Freiraum 23 gebildet. Der erste Schenkel 21 ist durch eine geringere Materialstärke als Federzunge ausgebildet und mithin relativ zu dem zweiten Schenkel 22 nach außen bewegbar gegen eine diesen Schenkel 21 in seine Ruheposition zurückdrängende Rückstellkraft. Zur Verringerung, idealerweise Ausräumung der Gefahr einer Rissbildung am Grund des Schlitzes 20, wenn der Schenkel 21 ausgelenkt wird, ist der Schlitz 20 an seinem Grund mit einer im Querschnitt kreisabschnittsförmigen Schlitzaufweitung 24 versehen.

Im Gegensatz zu dem ersten Schenkel 21 ist der zweite Schenkel 22 starr und nicht umbiegbar geformt.

An seinem offenen Ende ist durch eine Abschrägung 25 am freien Ende des ersten Schenkels 21 eine keilförmige Aufweitung des Schlitzes erreicht. Die Abschrägung ist dabei in einer solchen Weise geformt, dass bei einer Lage des Schiebers 4 in seiner proximalen Endstellung, wie dies in Fig. 3 gezeigt ist, der Verlauf dieser Abschrägung 25 im Wesentlichen senkrecht zu der Erstreckungsrichtung des Schaftelementes 3 liegt.

Während einer Schließ- bzw. Spreizbewegung der beiden Griffarme 6, 8 bewegt sich nun der Querstift 15 in dem Schlitz 20 hin und her und wird so mitgenommen, führt damit zu einer axialen Längsbewegung des Schiebers 4 relativ zu dem Schaftelement 3. Dabei wird für eine Bewegung in Richtung des distalen Endes 16 die Kraft von dem zweiten Griffarm 8 auf den Querstift 15 über den starren Schenkel 22 übertragen, so dass hier eine größere Kraft übertragen werden kann. Da diese Bewegungsrichtung des Schiebers 4 die Schließbewegung der Räumschneiden 17 und 18 bestimmt, kann also bei einem Schneidvorgang ein erhebliches Maß an Kraft übertragen werden. In der umgekehrten Richtung, der Öffnungsrichtung, wird die für das Bewegen des Schiebers 4 in Richtung des proximalen Endes 5 erforderliche Kraft über den als Federzunge ausgebildeten ersten Schenkel 21 auf den Querstift 15 übertragen. Typischerweise wird bei der Öffnungsbewegung der Räumschneiden 17, 18 dabei eine geringere Kraft benötigt, so dass hier keine Fehlfunktion auftritt, die durch eine nach außen gerichtete und gegen die Federkraft erfolgenden Ausweichbewegung des ersten Schenkels 21 geschehen könnte.

In der in Fig. 3 gezeigten Position, in der sich der Schieber 4 in seiner proximalen Endstellung befindet, ist zu erkennen, dass der erste Schenkel 21 den Querstift 15 noch mit einem Ende in einer Richtung senkrecht zur Längserstreckungsrichtung des Schaftelementes 3 überragt und in seiner Position sichert. Wird nun aber, z.B. mit dem Daumen einer Betätigungshand, eine Druckkraft auf den Ansatz 14 übertragen, so führt dies zu einer nach außen gerichteten und gegen die Federkraft des als Federzunge ausgebildeten ersten Schenkels 21 wirkenden Ausweichbewegung dieses ersten Schenkels 21, bis der Querstift 15 freigegeben ist. Dann kann der Schieber 4 von dem Schaftelement 3 abgehoben und entnommen werden.

Gegebenenfalls ist der Schieber 4 dann noch über die Räumschneide 18 mit dem Schaftelement 3 verbunden, was jedoch für die Reinigung dieser Elemente keine Schwierigkeiten darstellt. Falls erforderlich, kann für eine Reinigung auch noch eine entsprechende Trennung am distalen Ende 16 erfolgen, wobei die diesbezüglichen Mechanismen und Schritte abhängig sind von der Ausgestaltung des Werkzeuges, in an sich bekannter Weise stattfinden können.

In der in Fig. 3 gezeigten Position, in der der Schieber seine proximale Endstellung erreicht hat, liegt der zweite Griffarm 8 mit einer Innenfläche 26 an einer die Durchführung 19 begrenzenden Anschlagfläche 27 an, so dass auch beim Aufbringen einer Lösekraft auf den Ansatz 14 eine weitergehende Auswärtsbewegung des Schiebers 4 in Richtung des proximalen Endes so lange nicht möglich ist, bis der Querstift 15 aus dem Schlitz 20 gelöst ist.

Zum Zusammenfügen des erfindungsgemäßen Schiebeschaftinstrumentes 1 wird der zweite Griffarm 8 in die in Fig. 3 gezeigte Position geführt, die durch den Anschlag der Innenfläche 26 des Griffarmes 8 an der Anschlagfläche 27 definiert ist, und es wird der Schieber 4 in Richtung des Schaftelementes 3 geführt, so dass der Querstift 15 an der Abschrägung 25 des ersten Schenkels 21 vorbei in den Schlitz 20 geleitet wird und durch Aufbringen einer Druckkraft unter Abspreizen des ersten Schenkels 21 in diesen Schlitz einrastet. So ist dann die verbundene Position erreicht, kann eine in Richtung des distalen Endes gerichtete Kraftübertragung durch Schließen der Griffarme 6 und 8 ausgeübt werden.

Sowohl beim Lösen als auch beim Verbinden des Schiebers 4 vom bzw. mit dem Schaftelement 3 wird auch die Längsführung, wie sie näher in Bezug auf die hinsichtlich der Längsführung gleiche, in der Anbindung des zweiten Griffarms 8 an den Querstift 15 abweichende Ausführungsform nach den Figuren 4 bis 8 beschrieben ist, gelöst bzw. verbunden.

In den Figuren 4 bis 8 ist eine zweite Ausgestaltungsvariante der Anbindung zwischen dem zweiten Griffteil 8 und dem Schieber 4 bei einem Schiebeschaftinstrument 1 gemäß Fig. 1 gezeigt und wird nachfolgend näher erläutert.

Der grundsätzliche Aufbau des Schiebeschaftinstrumentes 1 in diesem zweiten Ausführungsbeispiel gleicht dem, wie er in Bezug auf das erste Ausführungsbeispiel und unter Einbeziehung der Figuren 1 bis 3 beschrieben worden ist. Insoweit kann hier hinsichtlich des Aufbaus des Schiebeschaftinstrumentes 1 und der Funktion der einzelnen Elemente auf die vorhergehende Beschreibung Bezug genommen und verwiesen werden. Dies gilt insbesondere dort, wo in den Figuren 4 bis 8 für die gleichen Elemente, wie sie auch in den Figuren 1 bis 3 dargestellt sind, die gleichen Bezugszeichen verwendet werden.

Der wesentliche Unterschied zwischen dem in den Figuren 4 bis 8 gezeigten zweiten Ausführungsbeispiel und dem zuvor beschriebenen ersten Ausführungsbeispiel besteht in der Gestaltung des oberen Anschlussendes des zweiten Griffteils 8, welches die Verbindung mit dem Schieber 4 über den Querstift 15 herstellt. Wie insbesondere in den Figuren 4 und 5 gut zu erkennen ist, ist auch bei diesem Ausführungsbeispiel am Anschlussende des Griffteils 8 ein Längsschlitz 20 vorgesehen, in welchem der Querstift 15 ruht und über welchen eine Kraftübertragung von dem zweiten Griffteil 8 auf den Schieber 4 zu dessen längsgeführter Bewegung entlang dem Schaftelement 3 erfolgt. Auch in diesem Ausführungsbeispiel ist der Schlitz 20 von einem ersten Schenkel 21 sowie einem zweiten Schenkel 22 begrenzt. Auch hier ist der erste Schenkel 21 als Federzunge ausgebildet mit einer Abschrägung 25 an seinem freien Ende, die ein einfacheres Einführen des Querstiftes 15 in den Schlitz 20 ermöglicht, wie dies bereits vorstehend anhand des vorhergehend beschriebenen Ausführungsbeispiels beschrieben ist. Der zweite Schenkel 22 ist auch in dieser Ausgestaltungsvariante ein starrer Schenkel, der auch dann nicht federnd nachgibt, wenn über ihn eine Kraft auf den Querstift 15 ausgeübt wird, um den Schieber 4 entlang des Schaftelementes 3 in Richtung des distalen Endes zu bewegen.

Abweichend von der Gestaltung in dem ersten Ausführungsbeispiel weist das Griffteil 8 an seinem oberen Ende zusätzlich einen zweiten Schlitz 29 auf, der sich an die dem ersten Schlitz 20 gegenüberliegende Seite des ersten Schenkels 21 anschließt und auf der anderen Seite des Schlitzes von einem dritten Schenkel 28 begrenzt ist. Dabei verlaufen die Schlitze 20 und 29 im Wesentlichen parallel, so dass sich ein zwischen diesen stehender erster Schenkel 21 mit im Wesentlichen gleichbleibender Materialstärke in dieser Richtung ergibt. Zudem sind beide Schlitze 20 und 29 gleich tief in das Material des freien Endes des Griffteils 8 eingeführt bzw. eingeschnitten, so dass sie eine beidseits auf gleichem Niveau liegende Anbindung des unteren Endes des ersten Schenkels 21 an das darunter liegende Material des Griffteils 8 ergeben.

Der dritte Schenkel 28 ist erneut ein starrer, d.h. ein solcher, der bei einer im üblichen Umgang mit dem Schiebeschaftinstrument 1 auf ihn aufgebrachten Kraft nicht verformt und federnd ausweicht.

In den Figuren 4 und 5 ist dabei einmal (Fig. 4) der Ausschnitt aus dem Schiebeschaftinstrument gemäß dem zweiten Ausführungsbeispiel mit einer Position des Schiebers 4 in einer maximal in Richtung des distalen Endes verschobenen Stellung auf dem Schaftelement 3 und in Fig. 5 in umgekehrter Richtung in einer maximal in Richtung des proximalen Endes 5 verschobenen Position des Schiebers 4 gezeigt. Insoweit gleichen diese Figuren 4 und 5 in ihren Positionen und Darstellungen den Figuren 2 und 3, so dass hier besonders gut die Unterschiede zwischen den Ausführungsbeispielen erkennbar sind.

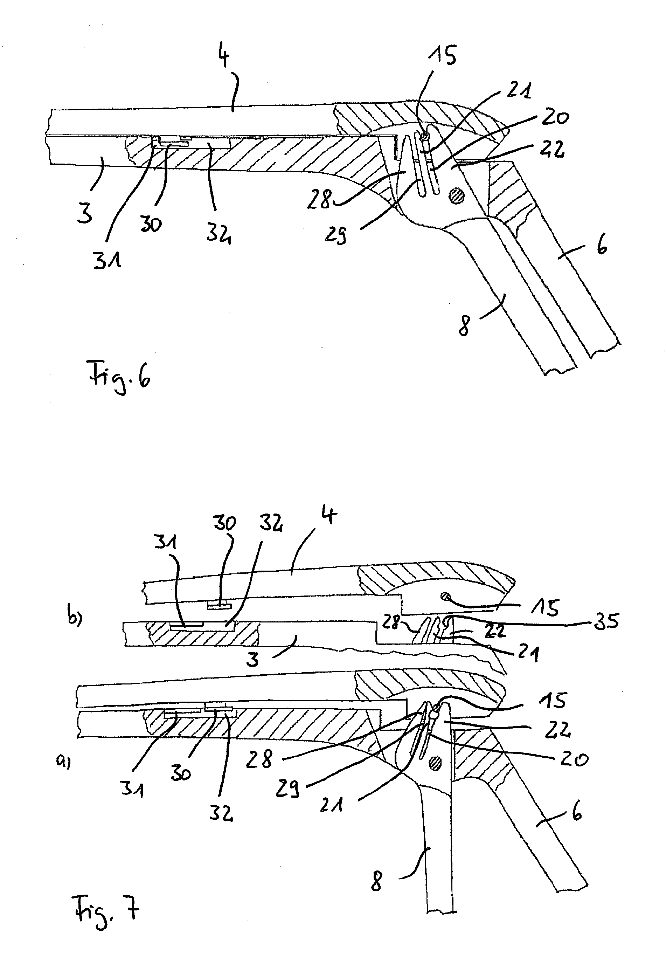

Weiterhin sind in den Darstellungen der Figuren 6 bis 8 diejenigen Strukturen dargestellt, die für eine Längsführung des Schiebers 4 an dem Schaftelement 3 sorgen und zugleich die Verbindung quer zu dieser Längsrichtung halten. Diese Strukturen, die im Zusammenhang mit der Darstellung des Schiebeschaftinstrumentes 1 nach dem zweiten Ausführungsbeispiel explizit dargestellt sind und nachfolgend näher beschrieben werden, sind in gleicher Weise auch bei dem ersten Ausführungsbeispiel gemäß den Figuren 1 bis 3 vorhanden und an den einzelnen Elementen so ausgebildet. Insoweit kann die nachfolgende Beschreibung ebenso auch für das voranstehende erste Ausführungsbeispiel hergenommen werden, da sie auch für dieses gleichermaßen gilt.

Zu erkennen ist, dass auf der bei zusammengesetztem Schiebeschaftinstrument 1 dem Schieber 4 zugewandten Oberfläche 33 eine in Längsrichtung des Schaftelementes 3 verlaufende Führungsnut 31 angeordnet ist. Die Führungsnut 31 weist eine gegenüber dem oberflächennahen Schlitz verbreiterten Nutengrund auf, so dass sich seitlich überstehende Führungs- und Haltestege ergeben. An ihren proximal gelegenen Ende ist die Führungsnut mit einer Nutenaufweitung 32 versehen, in der die überstehenden Führungsstege ausgeräumt, die Breite des Schlitzes an die Breite des Nutengrundes angepasst und also verbreitert ist.

Weiterhin ist auf einer Oberfläche 34 des Schiebers 4, die im zusammengesetzten Zustand des Schiebeschaftinstrumentes 1 der Oberfläche 33 des Schaftelementes 3 zugewandt ist, ein von dieser Oberfläche vorstehender Nutenstein 30 fest angeformt. Dieser verfügt über einen schmalen Verbindungssteg zu der Oberfläche 34 und hat einen seitlich verbreiterten Fuß. Die Breite des Fußes des Nutensteines 30 ist dabei so bemessen, dass der Nutenstein 30 in die Nutenaufweitung 32 eingesetzt werden kann. Wird der Schieber 4 dann weiter vorgeschoben in Richtung des distalen Endes, gleitet der Fuß des Nutensteines 30 entlang des Nutengrundes, und der Steg fährt in den Schlitz der Führungsnut 31 ein. So wird dann der Nutenstein 30 in der Führungsnut 31 aufgrund des Zusammenwirkens der Führungs- und Haltestege mit dem Fuß des Nutensteines 30 zurückgehalten gegen etwaige Trennbewegungen quer zur Längserstreckung von Schieber 4 und Schaftelement 3.

Die Situation des Einsetzens bzw. Trennens des Nutensteines 30 in die Nutenaufweitung 32 bzw. Führungsnut 31 bzw. aus diesen heraus ist in der Fig. 7 in den Darstellungen a und b veranschaulicht. Dort ist gut zu sehen, wie der Nutenstein 30 (vgl. Fig. 7a) in die Nutenaufweitung passt und dort eingeführt werden kann. In der Fig. 7b ist die Situation nach der Trennung der Elemente Schieber 4 und Schaftelement 3 dargestellt bzw. kurz vor dem Zusammensetzen.

Die Fig. 7 enthält weiterhin eine Veranschaulichung der Wirkweise des dritten Schenkels 28, der beim Einführen des Querstiftes 15 in den Schlitz 20, in einer Situation also, in der der als Federzunge ausgebildete erste Schenkel 21 unter Aufweitung des Schlitzes 20 von dem zweiten Schenkel 22 weg bewegt wird, insbesondere an seinem freien Ende, einen Anschlag für das freie Ende des ersten Schenkels 21 bildet. In der in Fig. 7a dargestellten Situation stößt das freien Ende des ersten Schenkels 21 an dem freien Ende des dritten Schenkels 28 an, so dass ein weiteres Ausweichen des ersten Schenkels 21 nicht mehr möglich ist. Auf diese Weise wird ein Überspreizen des Schlitzes 20 verhindert, so dass keine Gefahr einer Beschädigung des ersten Schenkels 21 oder einer Rissbildung am Grund des Schlitzes 20 besteht. Gut zu erkennen ist hier ebenfalls, dass beim Ausweichen des als Federzunge gebildeten ersten Schenkels 21 zum Öffnen des Schlitzes 20 der weitere Schlitz 29 in seiner Breite verringert, bei einem Anschlag des ersten Schenkels 21 am dritten Schenkel 28 dort vollständig geschlossen wird.

Bei dem zweiten gezeigten Ausführungsbeispiel weist der erste Schlitz 20 in einem oberen Abschnitt eine im Radius in etwa an den Durchmesser des Querstiftes 25 angepasste kreisförmige Aufweitung 35 auf. Diese bildet eine Art Haltemulde und dient der Definition einer Ruheposition des Querstiftes 15, in welcher dieser nach dem Einsetzen in den Schlitz 20 ruht und je nach Materialbeschaffenheit der Schenkel 21, 22 ggf. auch verrasten kann. Insbesondere dann, wenn während der Bewegung des Schiebers 4 in Richtung des distalen Endes zum Schließen der beiden Räumschneiden 17 und 18 der Querstift 15 in dem Schlitz 20 in Richtung seines Schlitzgrundes, also in Richtung des freien Endes des Griffabschnittes 8 wandert, wird dies in dem gezeigten Ausführungsbeispiel erneut unter einem Öffnen des Schlitzes 20, also unter einem federnden Ausweichen des ersten Schenkels 21, geschehen. In der distalen Endposition, so ist das Schiebeschaftinstrument 1 konstruiert, ruht der Querstift 15 wiederum in der kreisförmigen Aufweitung 35 und wird dort durch die Federwirkung des als Federzunge ausgebildeten ersten Schenkels 21 gehalten. Auf dieser Weise sind zwei Positionen gebildet, in denen das Schiebeschaftinstrument 1 in gewisser Weise festgelegt werden, ja nach Ausgestaltung der Schenkel 21, 22 auch verrasten kann, nämlich in der distalen Endposition, in der die Räumschneiden 17, 18 geschlossen sind, und in einer proximalen Endposition, in der die Räumschneiden 17, 18 geöffnet sind und der Schieber durch Aufbringung auf die Andruckfläche 14, wie dies oben am Beispiel des ersten Ausführungsbeispiels ausführlich beschrieben ist und auch für das zweite Ausführungsbeispiel zu praktizieren ist, gelöst werden kann. Diese zusätzlichen Haltepositionen sind insbesondere günstig, da bei in der distalen Endposition gehaltenem Schieber 4 die Räumschneiden 17, 18 geschlossen bleiben, mithin mit dem Schiebeschaftinstrument 1 zu entfernendes organisches Material unterstützt durch diesen Haltemechanismus zusätzlich festgehalten wird, ohne dass ein Operateur beim Herausziehen des Schiebeschaftinstrumentes aus dem Arbeitsbereich dieses in der distalen Position mit ansonsten erforderlicher höherem Kraftaufwand halten müsste, um das zu entfernende organische Material nicht etwa auf dem Weg durch den Körper loszulassen und zu verlieren.

Eine derartige kreisförmige Aufweitung 35, wie sie in dem zweiten Ausführungsbeispiel dargestellt ist, ist auch für das erste Ausführungsbeispiel vorgesehen.

Auch aus der voranstehenden Beschreibung der Ausführungsbeispiele ist noch einmal deutlich geworden, welche Vorteile die erfindungsgemäße Ausgestaltung des neuartigen Schiebeschaftinstrumentes mit sich bringt, indem insbesondere Verriegelungsmechanismen und entsprechende Teile entfallen können, was nicht nur die Bedienung des Schiebeschaftinstrumentes im Einsatz wie auch beim Zerlegen bzw. Zusammenfügen erleichtert, sondern auch eine Erleichterung beim Reinigen und Sterilisieren ergibt.

### Bezugszeichenliste

- 1: Schiebeschaftinstrument
- 2: Schaft
- 3: Schaftelement
- 4: Schieber
- 5: proximales Ende
- 6: erster Griffarm
- 7: Fingeröse
- 8: zweiter Griffarm
- 9: Schwenklagerstift
- 10: Stiftaufnahme
- 11: Fingeröse
- 12: Fingermulde
- 13: Fortsatz
- 14: Ansatz
- 15: Querstift
- 16: distales Ende
- 17: Räumschneide
- 18: Räumschneide
- 19: Durchführung
- 20: Schlitz
- 21: erster Schenkel
- 22: zweiter Schenkel
- 23: Ausnehmung
- 24: Schlitzaufweitung
- 25: Abschrägung
- 26: Innenfläche
- 27: Anschlagfläche
- 28: dritter Schenkel
- 29: Schlitz
- 30: Nutenstein
- 31: Führungsnut
- 32: Nutenaufweitung
- 33: Oberfläche
- 34: Oberfläche
- 35: kreisförmige Aufweitung

## Patentansprüche

1. Medizinisches, insbesondere chirurgisches, Schiebeschaftinstrument (1) mit einem ersten Griffarm (6) und einem in Bezug auf den ersten Griffarm (6) schwenkbar gelagerten zweiten Griffarm (8), mit einem mit dem ersten Griffarm (6) verbundenen Schaftelement (3) und einem an dem Schaftelement (3) axial verschiebbaren, mit dem zweiten Griffarm (8) kuppelbaren Schieber (4), wobei der Schieber (4) in einer zurückgezogenen proximalen Endstellung seines Schiebeweges von dem Schaftelement (3) abnehmbar ist und in von der proximalen Endstellung in Richtung einer vorgeschobenen distalen Endstellung verschobenen weiteren Bereichen seines Schiebeweges untrennbar an dem Schaftelement (3) geführt ist und wobei zur Kupplung des zweiten Griffarms (8) mit dem Schieber (4) ein an dem Schieber (4) angeordneter Querstift (15) in einem endseitig offenen, von zwei Schenkeln (21, 22) begrenzten ersten Schlitz (20) des zweiten Griffarms (8) aufgenommen ist, und wobei an dem proximalen Ende des Schiebers (4) ein Ansatz (14) angeordnet ist, der in der proximalen Endstellung über ein proximales Ende des Schaftelementes (3) hinausragt und über den eine Lösekraft auf den Schieber (4) aufbringbar ist, **dadurch gekennzeichnet, dass** ein erster Schenkel (21) der den ersten Schlitz (20) begrenzenden Schenkel (21, 22) als quer zu seiner Längserstreckungsrichtung von dem zweiten der Schenkel (22) nach außen weg bewegbare Federzunge gebildet ist, wobei in der proximalen Endstellung der zweite Griffarm (8) derart positioniert ist, dass der Querstift (15) in einer im wesentlichen senkrecht von der axialen Linie des Schaftelements (3) in Richtung des Schiebers (4) weisenden Löserichtung von dem ersten als Federzunge gebildeten Schenkel (21) zumindest teilweise überdeckt und zurückgehalten ist, durch Aufbringen einer Lösekraft über den Ansatz (14), in der Löserichtung unter gegen die Federkraft der Federzunge gerichteten Verlagerung des Endes des ersten Schenkels (21) aus dem Schlitz (20) lösbar ist.

2. Schiebeschaftinstrument nach Anspruch 1, **gekennzeichnet durch** eine V- oder keilförmige Aufweitung (25) des ersten Schlitzes (20) an seinem offenen Ende.

3. Schiebeschaftinstrument nach einem der vorhergehenden Ansprüche, **gekennzeichnet durch** eine am Grund des ersten Schlitzes (20) zwischen den Schenkeln (21, 22) gebildete, insbesondere kreisabschnittförmige Schlitzaufweitung (24).

4. Schiebeschaftinstrument nach einem der vorhergehenden Ansprüche, **dadurch gekennzeichnet, dass** der zweite Schenkel (22) ein starrer Schenkel ist.

5. Schiebeschaftinstrument nach einem der vorhergehenden Ansprüche, **dadurch gekennzeichnet, dass** der zweite Schenkel (22) beim Betätigen der Griffarme (6, 8) in eine erste Betätigungsrichtung auf den Querstift (15) eine Kraft zum Bewegen des Schiebers (4) in Richtung der distalen Endstellung überträgt, während der erste Schenkel (21) beim Betätigen der Griffarme (6, 8) in eine zweite Betätigungsrichtung auf den Querstift (15) eine Kraft zum Bewegen des Schiebers (4) in Richtung der proximalen Endstellung überträgt.

6. Schiebeschaftinstrument nach einem der vorhergehenden Ansprüche, **dadurch gekennzeichnet, dass** in die Oberfläche des zweiten Griffarms (8) auf dessen dem ersten Griffarm (6) zugewandten Seite eine Fingermulde (12) eingeformt ist.

7. Schiebschaftinstrument nach einem der vorhergehenden Ansprüche, **dadurch gekennzeichnet, dass** an den freien Enden der Griffarme (6, 8) jeweils eine Fingeröse (7, 11) ausgebildet ist.

8. Schiebeschaftinstrument nach einem der vorhergehenden Ansprüche, **dadurch gekennzeichnet, dass** der zweite Griffarm (8) lösbar mit dem ersten Griffarm (6) und/oder dem Schaftelement (3) verbunden ist.

9. Schiebeschaftinstrument nach einem der vorhergehenden Ansprüche, **dadurch gekennzeichnet, dass** an dem distalen Ende des Schaftelementes (3) eine erste feststehende Schneide (17) ausgebildet ist und dass ferner eine relativ zu der ersten Schneide (17) bewegbare zweite Schneide (18) angeordnet ist, die mit dem Schieber (4) derart gekoppelt ist, dass sich bei einer Verlagerung des Schiebers (4) in axialer Richtung die Schneiden (17, 18) aufeinander zu bzw. von einander weg bewegen.

10. Schiebeschaftinstrument nach einem der vorhergehenden Ansprüche, **gekennzeichnet durch** einen zweiten endseitig offenen Schlitz (29) im Bereich des ersten Schlitzes (20), der sich zwischen dem ersten Schenkel (21) und einem dritten Schenkel (28) erstreckt, wobei der erste Schenkel (21), wenn dieser quer zu seiner Längserstreckungsrichtung von dem zweiten Schenkel (22) nach außen weg bewegt wird, sich zugleich auf den dritten Schenkel (28) zu bewegt und der dritte Schenkel (28) einen den Bewegungsweg des ersten Schenkels (21) begrenzenden Anschlag bildet.

11. Schiebeschaftinstrument nach Anspruch 10, **dadurch gekennzeichnet, dass** der zweite Schlitz (29) parallel zu dem ersten Schlitz (20) verläuft und sich im Wesentlichen über eine gleiche Länge in das Material des zweiten Griffarms (8) hinein erstreckt wie der ersten Schlitz (20).

12. Schiebeschaftinstrument nach einem der Ansprüche 10 oder 11, **dadurch gekennzeichnet, dass** der dritte Schenkel (28) ein starrer Schenkel ist.

13. Schiebeschaftinstrument nach einem der vorhergehenden Ansprüche, **dadurch gekennzeichnet, dass** der Längsschlitz in einem seinem freien, offenen Ende nahegelegenen Bereich eine kreisförmige Aufweitung (35) aufweist, deren Durchmesser dem Durchmesser des Querstifts (15) entspricht.

## Claims

1. Medical, in particular surgical, sliding shaft instrument (1) with a first grip arm (6) and a second grip arm (8) mounted pivotably in relation to the first grip arm (6), with a shaft element (3) connected to the first grip arm (6), and with a slider (4) which can be coupled to the second grip arm (8) and which is axially displaceable on the shaft element (3), wherein the slider (4), in a retracted proximal end position of its sliding path, can be removed from the shaft element (3) and, in other areas of its sliding path located away from the proximal end position in the direction of an advanced distal end position, is guided inseparably on the shaft element (3), and wherein, in order to couple the second grip arm (8) to the slider (4), a transverse pin (15) arranged on the slider (4) is received in a first slot (20) of the second grip arm (8), which slot (20) is open at one end and is defined by two limbs (21, 22), and wherein a lug (14) is arranged at the proximal end of the slider (4), which lug (14), in the proximal end position, protrudes beyond a proximal end of the shaft element (3) and serves for applying a release force to the slider (4), **characterized in that** a first limb (21) of the limbs (21, 22) defining the first slot (20) is formed as a resilient tongue which is movable, transversely with respect to the direction of its longitudinal extent, outwards away from the second of the limbs (22), wherein the second grip arm (8), in the proximal end position, is positioned in such a way that the transverse pin (15), in a release direction facing substantially perpendicularly from the axial line of the shaft element (3) in the direction of the slider (4), is at least partially covered and held back by the first limb (21) formed as a resilient tongue, and, by applying a release force via the lug (14) in the release direction, can be released from the slot (20) by displacing the end of the first limb (21) counter to the spring force of the resilient tongue.

2. Sliding shaft instrument according to Claim 1, **characterized by** a V-shaped or wedge-shaped widening (25) of the first slot (20) at the open end thereof.

3. Sliding shaft instrument according to one of the preceding claims, **characterized by** a slot widening (24), in particular in the shape of a segment of a circle, formed at the bottom of the first slot (20) between the limbs (21, 22).

4. Sliding shaft instrument according to one of the preceding claims, **characterized in that** the second limb (22) is a rigid limb.

5. Sliding shaft instrument according to one of the preceding claims, **characterized in that**, when the grip arms (6, 8) are actuated in a first actuation direction, the second limb (22) transmits to the transverse pin (15) a force for moving the slider (4) in the direction of the distal end position, whereas, when the grip arms (6, 8) are actuated in a second actuation direction, the first limb (21) transmits to the transverse pin (15) a force for moving the slider (4) in the direction of the proximal end position.

6. Sliding shaft instrument according to one of the preceding claims, **characterized in that** a finger groove (12) is formed into the surface of the second grip arm (8), on the side thereof facing towards the first grip arm (6).

7. Sliding shaft instrument according to one of the preceding claims, **characterized in that** in each case a finger ring (7, 11) is formed at the free ends of the grip arms (6, 8).

8. Sliding shaft instrument according to one of the preceding claims, **characterized in that** the second grip arm (8) is releasably connected to the first grip arm (6) and/or to the shaft element (3).

9. Sliding shaft instrument according to one of the preceding claims, **characterized in that** a first, stationary blade (17) is formed at the distal end of the shaft element (3), and **in that** a second blade (18), movable relative to the first blade (17), is moreover provided which is coupled to the slider (4) in such a way that, when the slider (4) is displaced in the axial direction, the blades (17, 18) move towards each other or away from each other.

10. Sliding shaft instrument according to one of the preceding claims, **characterized by** a second slot (29), open at one end, in the area of the first slot (20), which second slot (29) extends between the first limb (21) and a third limb (28), wherein the first limb (21), when moved outwards transversely with respect to the direction of its longitudinal extent away from the second limb (22), moves at the same time towards the third limb (28), and the third limb (28) forms an abutment that limits the path of movement of the first limb (21).

11. Sliding shaft instrument according to Claim 10, **characterized in that** the second slot (29) runs parallel to the first slot (20) and extends into the material of the second grip arm (8) by substantially the same length as the first slot (20).

12. Sliding shaft instrument according to one of Claims 10 and 11, **characterized in that** the third limb (28) is a rigid limb.

13. Sliding shaft instrument according to one of the preceding claims, **characterized in that** the longitudinal slot has, in an area located near its free open end, a circular widening (35), of which the diameter corresponds to the diameter of the transverse pin (15).

## Revendications

1. Instrument médical, en particulier chirurgical, à tige coulissante (1), comprenant un premier bras de préhension (6) et un deuxième bras de préhension (8) monté de manière pivotante par rapport au premier bras de préhension (6), comprenant un élément de tige (3) connecté au premier bras de préhension (6) et un élément coulissant (4) déplaçable axialement sur l'élément de tige (3), pouvant être accouplé au deuxième bras de préhension (8), l'élément coulissant (4) pouvant être enlevé de l'élément de tige (3) dans une position d'extrémité proximale rétractée de sa course de coulissement et étant guidé de manière non séparable sur l'élément de tige (3) dans d'autres régions de sa course de coulissement déplacées de la position d'extrémité proximale dans la direction d'une position d'extrémité distale avancée, et, pour l'accouplement du deuxième bras de préhension (8) à l'élément coulissant (4), une goupille transversale (15) disposée au niveau de l'élément coulissant (4) étant reçue dans une première fente (20) du deuxième bras de préhension (8) ouverte du côté de l'extrémité, limitée par deux branches (21, 22), et une partie saillante (14) étant disposée au niveau de l'extrémité proximale de l'élément coulissant (4), laquelle fait saillie dans la position d'extrémité proximale au-delà d'une extrémité proximale de l'élément de tige (3) et par le biais de laquelle une force de desserrage peut être appliquée à l'élément coulissant (4), **caractérisé en ce qu'**une première branche (21) des branches (21, 22) limitant la première fente (20) est formée en tant que langue élastique déplaçable transversalement à sa direction d'étendue longitudinale vers l'extérieur à l'écart de la deuxième des branches (22), le deuxième bras de préhension (8), dans la position d'extrémité proximale, étant positionné de telle sorte que la goupille transversale (15) soit au moins en partie recouverte et retenue par la première branche (21) formée en tant que langue élastique dans une direction de desserrage orientée essentiellement perpendiculairement à l'orientation axiale de l'élément de tige (3) dans la direction de l'élément coulissant (4), et puisse être desserrée par l'application d'une force de desserrage par le biais de la partie saillante (14) dans la direction de desserrage, par décalage, orienté dans le sens opposé à la force de ressort de la langue élastique, de l'extrémité de la première branche (21) hors de la fente (20).

2. Instrument à tige coulissante selon la revendication 1, **caractérisé par** un élargissement en forme de V ou en forme de coin (25) de la première fente (20) au niveau de son extrémité ouverte.

3. Instrument à tige coulissante selon l'une quelconque des revendications précédentes, **caractérisé par** un élargissement de la fente (24), notamment en forme de section circulaire, formé à la base de la première fente (20) entre les branches (21, 22).

4. Instrument à tige coulissante selon l'une quelconque des revendications précédentes, **caractérisé en ce que** la deuxième branche (22) est une branche rigide.

5. Instrument à tige coulissante selon l'une quelconque des revendications précédentes, **caractérisé en ce que** la deuxième branche (22), lors de l'actionnement des bras de préhension (6, 8) dans une première direction d'actionnement, transfère à la goupille transversale (15) une force pour déplacer l'élément coulissant (4) dans la direction de la position d'extrémité distale, tandis que la première branche (21), lors de l'actionnement des bras de préhension (6, 8) dans une deuxième direction d'actionnement, transfère à la goupille transversale (15) une force pour déplacer l'élément coulissant (4) dans la direction de la position d'extrémité proximale.

6. Instrument à tige coulissante selon l'une quelconque des revendications précédentes, **caractérisé en ce que** dans la surface du deuxième bras de préhension (8) sur son côté tourné vers le premier bras de préhension (6) est façonné un creux pour les doigts (12).

7. Instrument à tige coulissante selon l'une quelconque des revendications précédentes, **caractérisé en ce qu'**un oeillet pour les doigts (7, 11) est à chaque fois réalisé au niveau des extrémités libres des bras de préhension (6, 8).

8. Instrument à tige coulissante selon l'une quelconque des revendications précédentes, **caractérisé en ce que** le deuxième bras de préhension (8) est connecté de manière desserrable au premier bras de préhension (6) et/ou à l'élément de tige (3).

9. Instrument à tige coulissante selon l'une quelconque des revendications précédentes, **caractérisé en ce qu'**un premier tranchant fixe (17) est réalisé au niveau de l'extrémité distale de l'élément de tige (3) et **en ce qu'**en outre un deuxième tranchant (18) est disposé de manière déplaçable par rapport au premier tranchant (17), lequel est accouplé à l'élément coulissant (4) de telle sorte que dans le cas d'un déplacement de l'élément coulissant (4) dans la direction axiale, les tranchants (17, 18) se déplacent l'un vers l'autre ou à l'écart l'un de l'autre.

10. Instrument à tige coulissante selon l'une quelconque des revendications précédentes, **caractérisé par** une deuxième fente ouverte du côté de l'extrémité (29) dans la région de la première fente (20), laquelle s'étend entre la première branche (21) et une troisième branche (28), la première branche (21), lorsque celle-ci est déplacée vers l'extérieur transversalement à sa direction d'étendue longitudinale à l'écart de la deuxième branche (22), se déplaçant en même temps vers la troisième branche (28) et la troisième branche (28) formant une butée limitant la course de déplacement de la première branche (21).

11. Instrument à tige coulissante selon la revendication 10, **caractérisé en ce que** la deuxième fente (29) s'étend parallèlement à la première fente (20) et s'étend essentiellement sur la même longueur à l'intérieur du matériau du deuxième bras de préhension (8) que la première fente (20).

12. Instrument à tige coulissante selon l'une quelconque des revendications 10 ou 11, **caractérisé en ce que** la troisième branche (28) est une branche rigide.

13. Instrument à tige coulissante selon l'une quelconque des revendications précédentes, **caractérisé en ce que** la fente longitudinale présente, dans une région proche de son extrémité libre ouverte, un élargissement de forme circulaire (35) dont le diamètre correspond au diamètre de la goupille transversale (15).
